# EUROPÄISCHE PATENTANMELDUNG

(11) **EP 1 894 555 A2**
(43) Veröffentlichungstag der Anmeldung: **05.03.2008**
(21) Anmeldenummer: 07015156.8
(22) Anmeldetag: 02.08.2007
(51) Int. Cl.: A61J 1/00

(54) **Vorrichtung zur Entnahme von Flüssigkeiten**

(30) Priorität: 30.08.2006 DE 102006040670
(71) Anmelder: Transcoject GmbH & Co. KG., 24539 Neumünster (DE)
(72) Erfinder: Heinz, Jochen, Dr., 24220 Flintbek (DE); Wildenhahn, Florian, 24114 Kiel (DE)
(74) Vertreter: Hemmer, Arnd

(57) **Zusammenfassung**

Eine Entnahmevorrichtung zur Entnahme von Flüssigkeit aus einem medizinischen Behälter weist ein Anschlussteil auf, welches zum Anschluss an den Behälter und zur Herstellung einer Leitungsverbindung in den Behälter ausgebildet ist. Weiter ist an der Entnahmevorrichtung ein zu dem Anschlussteil beweglich gelagertes Bauteil vorgesehen, das eine Vielzahl von Anschlüssen für einen Flüssigkeitsauslass aufweist, wobei das Anschlussteil und das Bauteil so zueinander bewegbar sind, dass eine Leitungsverbindung zwischen Behälter und wahlweise einem der Anschlüsse gebildet ist.

## Beschreibung

Die Erfindung betrifft eine Vorrichtung zur Entnahme von Flüssigkeit aus einem medizinischen Behälter mit den im Oberbegriff des Anspruchs 1 angegebenen Merkmalen.

Zur Entnahme von Flüssigkeiten aus medizinischen Behältern wie Infusionsflaschen, Kontrastmittelbehältern oder Ähnlichem ist es üblich, die von Gummimembranen gebildeten Verschlüsse dieser Behälter direkt mit Injektionsspritzen zu durchstechen und so den Behälterinhalt über eine Schlauchleitung einem Patienten zuzuführen. Daneben sind Anschlussadapter mit einem Hohldorn zum Durchstechen der Membran und mit einem Luerlockanschluss zum Anschließen einer Schlauchleitung bekannt, wobei an dem anderen Ende des Schlauches ein weiterer Luerlockanschluss vorgesehen ist, an dem eine Injektionsspritze anschließbar ist. Nachteilig an diesen Anschlussadaptern ist, dass sie nur zum einmaligen Gebrauch geeignet sind, da ansonsten nicht auszuschließen ist, dass Keime und andere Verunreinigungen über den Schlauch und insbesondere den Schlauchanschluss in den Behälter gelangen können. Zur mehrmaligen Entnahme aus den Flaschen werden diese deswegen nacheinander mit mehreren dieser Adapter durchstochen, was wiederum zum Nachteil hat, dass sich Gummipartikel bilden und verschleppen, sowie dass die mehrfach durchstochene Gummimembran undicht wird.

Vor diesem Hintergrund liegt der Erfindung die Aufgabe zugrunde, eine Vorrichtung zur Entnahme von Flüssigkeit aus einem medizinischen Behälter zu schaffen, welche zum mehrmaligen Gebrauch geeignet ist, wobei eine Verunreinigung des Behälters insbesondere durch Keime oder eine Beschädigung des Behälterverschlusses infolge von Gummiverschleiß zuverlässig ausgeschlossen werden und die Entnahmevorrichtung in einfacher Weise herstellbar und bedienbar sein soll.

Diese Aufgabe wird erfindungsgemäß durch eine Entnahmevorrichtung mit den im Anspruch 1 angegebenen Merkmalen gelöst. Vorteilhafte Weiterbildungen der Erfindung ergeben sich aus den Unteransprüchen, der nachfolgenden Beschreibung und der Zeichnung.

Die erfindungsgemäße Vorrichtung zur Entnahme von Flüssigkeit aus einem medizinischen Behälter weist ein Anschlussteil auf, welches zum Anschluss an den Behälter und zur Herstellung einer Leitungsverbindung in den Behälter ausgebildet ist. Daneben weist die Entnahmevorrichtung ein Bauteil auf, welches zu dem Anschlussteil beweglich gelagert ist und eine Vielzahl von Anschlüssen für einen Flüssigkeitsauslass aufweist. Erfindungsgemäß sind das Anschlussteil und das Bauteil so zueinander bewegbar, dass eine Leitungsverbindung zwischen dem Behälter und wahlweise zumindest einem der Anschlüsse gebildet ist.

Die Entnahmevorrichtung ist typischerweise zum Anschluss an solche medizinische Behälter vorgesehen, bei denen an einem in Entnahmestellung an der Unterseite des Behälters angeordneten Membran, welche bei derartigen Behältern üblicherweise mittels einer Aluminiummanschette festgelegt ist, durch Perforation eine Entnahmeöffnung geschaffen wird, durch die die in dem Behälter befindliche Flüssigkeit schwerkraftbedingt abfließen kann. Dementsprechend hängt die Entnahmevorrichtung im montierten Zustand unterhalb des medizinischen Behälters. Aus Gewichtsgründen besteht die Vorrichtung daher zweckmäßig im Wesentlichen aus einem Kunststoffmaterial oder ähnlich leichten, dabei aber formbeständigen Materialien.

Die Befestigung der Entnahmevorrichtung an dem Behälter erfolgt über das Anschlussteil, welches zur Bildung einer form- und/oder kraftschlüssigen Verbindung mit dem Behälter ausgebildet sein kann. Eine bevorzugte Ausgestaltung des Anschlussteils ermöglicht ein Befestigen der Entnahmevorrichtung an dem Behälter durch ein Umgreifen der zum Festlegen der Membran vorgesehenen Aluminiummanschette.

Daneben ist ein Teil, typischerweise ein Hohldorn, des Anschlussteils zum Durchstoßen der Membran an dem Behälter ausgebildet, wobei dieses Teil auch die Leitungsverbindung von dem Behälter zu dem Anschlussteil herstellt und im montierten Zustand der Entnahmevorrichtung in den Behälter hineinragt.

Erfindungsgemäß ist der Flüssigkeitsauslass der Entnahmevorrichtung nicht an dem Anschlussteil vorgesehen, sondern erfolgt wahlweise über einen oder mehrere der Anschlüsse des Bauteils, welches relativ zu dem Anschlussteil bewegbar ist. Hierbei sind das Anschlussteil und das Bauteil zueinander so bewegbar, dass eine Leitungsverbindung zwischen dem Behälterinneren und zumindest einem der Anschlüsse, d. h. einem Flüssigkeitsauslass der Vorrichtung gebildet wird. Außerdem ermöglicht die Entnahmevorrichtung eine Relativbewegung von Bauteil und Anschlussteil in zumindest eine solche Position, in welcher keine fluidleitende Verbindung zwischen dem Behälterinneren und dem Flüssigkeitsauslass der Entnahmevorrichtung besteht, so dass mit dem Bauteil die Leitungsverbindung innerhalb der Entladevorrichtung auch verschließbar ist.

Entsprechend der Anzahl der jeweils einen Flüssigkeitsauslass bildenden Anschlüsse an dem Bauteil sind mit der Entnahmevorrichtung mehrere fluidleitende Verbindungen zwischen dem Behälter und einem Flüssigkeitsauslass herstellbar. Auf diese Weise ist vorteilhaft ein mehrmaliger Gebrauch der Entnahmevorrichtung möglich, ohne einen Flüssigkeitsauslass bzw. Anschluss der Entnahmevorrichtung mehr als einmal benutzen zu müssen. So kann das Anschlussteil beispielsweise bei einem ersten Gebrauch dieser Vorrichtung an einen medizinischen Behälter angekoppelt werden. Anschließend kann durch entsprechende Positionierung des Bauteils zu dem Anschlussteil eine Leitungsverbindung von dem Behälterinneren zu einem der Anschlüsse des Bauteils hergestellt werden und die in dem Behälter befindliche Flüssigkeit über einen an dem Anschluss angeschlossenen Schlauch zu einem Patienten oder Entnahmestelle weitergeleitet werden. Nach Beendigung der Flüssigkeitsentnahme können das Bauteil und das Anschlussteil so zueinander bewegt werden, dass das Bauteil die Leitungsverbindung innerhalb der Entnahmevorrichtung verschließt. Hierdurch ist es nicht erforderlich, die Entnahmevorrichtung bei Nichtbenutzung von dem Behälter abzukoppeln. Soll die Entnahmevorrichtung ein weiteres Mal benutzt werden, so werden das Anschlussteil und das Bauteil so zueinander weiter bewegt, dass eine Leitungsverbindung von dem Behälterinneren zu einem noch nicht benutzten Anschluss des Bauteils hergestellt wird. Dieser Vorgang kann entsprechend der Anzahl der an dem Bauteil vorgesehenen Anschlüsse mehrfach wiederholt werden.

In dem Anschlussteil ist das Bauteil vorteilhafterweise formschlüssig geführt und gehalten. So ist das Anschlussteil zur Aufnahme des Bauteils ausgebildet, wobei bevorzugt ein Teil des Anschlussteils einen Teil des Bauteils formschlüssig hintergreift. Daneben bildet das Anschlussteil eine Führung für das Bauteil, in welcher das Bauteil zur Herstellung einer Leitungsverbindung von dem Behälter zu einem Flüssigkeitsauslass bewegbar ist, wobei die Führung die Richtung der Relativbewegung von Anschlussteil und Bauteil vorgibt.

Die Anschlüsse an dem Bauteil sind zweckmäßig in Richtung der Relativbewegung von Anschlussteil und Bauteil in Reihe angeordnet. Diese Ausgestaltung gewährleistet, dass bei mehrmaliger Benutzung der Entnahmevorrichtung jeweils mittels einer Relativbewegung zwischen Anschlussteil und Bauteil nacheinander die Leitungsverbindungen von dem Behälterinneren zu einem Flüssigkeitsauslass gebildet werden, wobei der Flüssigkeitsauslass der jeweiligen Leitungsverbindungen von aufeinanderfolgenden Anschlüssen gebildet wird, welche an dem Bauteil in Bewegungsrichtung hintereinander angeordnet sind. Vorzugsweise sind an dem Anschlussteil und dem Bauteil Markierungen vorgesehen, die die Relativpositionen von Anschlussteil und Bauteil anzeigen, in welchen eine fluidleitende Verbindung von dem Behälterinneren zu einem an dem Bauteil angeordneten Anschluss besteht. Weiter vorteilhaft können das Anschlussteil und das Bauteil in den jeweils eine Leitungsverbindung zu einem Anschluss bildenden Stellungen eine Rastverbindung bilden, wobei beispielsweise ein an dem Anschlussteil vorgesehener Vorsprung in eine zu dem Vorsprung komplementäre Ausnehmung des Bauteils oder ein an dem Bauteil vorgesehener Vorsprung in eine entsprechende Ausnehmung an dem Anschlussteil einrastet.

In einer bevorzugten Ausführungsform der erfindungsgemäßen Entnahmevorrichtung ist das Bauteil kreisringförmig ausgebildet. Dabei sind die Flüssigkeitsauslässe bzw. die Anschlüsse über den Umfang des Kreisrings verteilt angeordnet. In diesem Fall ist das revolverförmige Bauteil an dem Anschlussteil um seine Mittelachse drehbar gelagert und ermöglicht in einfacher Weise das Drehen eines Flüssigkeitsauslasses, d.h. eines Anschlusses des Bauteils vor die in dem Anschlussteil angeordnete Fluidleitung und somit eine einfache Herstellung einer Leitungsverbindung von dem Behälter zu einem Flüssigkeitsauslass. Des Weiteren ermöglicht diese Ausgestaltung des Bauteils eine platzsparende Ausbildung der Entnahmevorrichtung.

Um einerseits zu verhindern, dass Keime und andere Verunreinigungen über die an dem Bauteil angeordneten Anschlüsse und das Anschlussteil in den medizinischen Behälter gelangen und anderseits eine Leckage im Bereich der Anbindung des Bauteils an dem Anschlussteil zu vermeiden, ist zwischen dem Anschlussteil und dem Bauteil vorteilhaft eine Dichtung vorgesehen, welche das Anschlussteil gegenüber dem Bauteil flüssigkeits- und keimdicht abdichtet. Diese Dichtung ist zweckmäßigerweise in dem Bereich der Entnahmevorrichtung vorgesehen, in welchem das Bauteil in dem Anschlussteil formschlüssig geführt und gehalten ist.

Insbesondere wenn das Bauteil kreisringförmig ausgebildet ist, wobei die Flüssigkeitsauslässe bzw. die Anschlüsse über den Umfang des Kreisrings verteilt angeordnet sind, kann eine solche Dichtung von einem Gummiring gebildet werden, der an einer Stirnfläche noppenförmige Erhöhungen aufweist, welche jeweils einen Strömungskanal bilden und in die Flüssigkeitsauslässe formschlüssig eingreifen. Dabei entsprechen die Kreisverteilung der noppenförmigen Erhöhungen und deren Anzahl der Kreisverteilung und Anzahl der an dem Bauteil angeordneten Anschlüsse.

Um mit der Entnahmevorrichtung eine Membran eines medizinischen Behälters durchstoßen zu können und eine Leitungsverbindung von dem Behälter zu der Entnahmevorrichtung zu schaffen, weist das Anschlussteil auf der vom Bauteil abgewandten Seite bzw. auf der dem Behälter zugewandten Seite einen Hohldorn auf.

Vorteilhaft sind bei der erfindungsgemäßen Entnahmevorrichtung zumindest zwei Anschlüsse vorgesehen, welche vorzugsweise verriegelbar ausgebildet sind und insbesondere sich hinsichtlich ihrer Form und/oder Abmessung unterscheiden. So können die Anschlüsse als Teil aller in der Medizintechnik zur Leitungsverbindung verwendeten Kupplungssysteme ausgebildet sein, wobei die Entnahmevorrichtung bei unterschiedlichen Anschlüssen beispielsweise den Anschluss von Schlauchleitungen mit unterschiedlichen Anschlussteilen ermöglicht.

In weiterer vorteilhafter Ausgestaltung sind bei der erfindungsgemäßen Entnahmevorrichtung die Anschlüsse an dem Bauteil, welche die Flüssigkeitsauslässe bilden, jeweils als Luerlockanschluss ausgebildet. Auf diese Weise können an den Anschlüssen in schneller und einfacher Weise Schlauchleitungen oder auch Spritzen mit korrespondierenden Luerlockanschlüssen angeschlossen werden.

Bevorzugt ist an jedem der Flüssigkeitsauslässe ein keimdichter und vorzugsweise nach dem Entfernen nicht wieder einsetzbarer Verschluss vorgesehen. Diese Verschlüsse sind zweckmäßigerweise derart ausgebildet, dass sie zumindest die Auslassöffnungen der Anschlüsse des Bauteils keimdicht abschließen. Eine Kontaminierung der Entnahmevorrichtung und damit des medizinischen Behälters mit Keimen und Verunreinigungen wird auf diese Weise verhindert. Weiter vorteilhaft werden bei Verwendung von Verschlüssen, welche nach dem Entfernen nicht wieder einsetzbar sind, die schon einmal benutzten Anschlüsse durch das Fehlen des Verschlusses kenntlich gemacht, um eine Mehrfachverwendung eines Anschlusses zu verhindern.

Vorzugsweise sind die Verschlüsse von einer Siegelfolie gebildet, welche die Auslassöffnungen der Anschlüsse keimdicht abdeckt. In weiterer bevorzugter Ausgestaltung ist vorgesehen, dass die Verschlüsse von Verschlusskappen gebildet sind.

Zur Vermeidung einer Mehrfachbenutzung eines Flüssigkeitsauslasses ermöglicht die erfindungsgemäße Entnahmevorrichtung bevorzugt nur eine Relativbewegung von Anschlussteil und Bauteil in eine Bewegungsrichtung. So können beispielsweise an dem Anschlussteil und dem Bauteil z. B. keilförmige Rastelemente vorgesehen sein, die derart ausgebildet sind, dass sie die Bewegung von Anschlussteil und Bauteil zueinander nur in einer festgelegten Richtung gewährleisten und eine entgegengesetzt gerichtete Bewegung blockieren. Bei einem Bauteil, bei welchem die Flüssigkeitsauslässe in endloser Folge, z.B. in kreisförmiger Anordnung vorgesehen sind kann des Weiteren mit an dem Anschlussteil und dem Bauteil angeordneten Anschlägen verhindert werden, dass Flüssigkeitsauslässe mehr als einmal zur Flüssigkeitsentnahme verwendet werden.

Nachfolgend wird die Erfindung anhand eines in der Zeichnung dargestellten Ausführungsbeispiels beschrieben. Darin zeigen:
- Fig. 1: in perspektivischer Explosionsdarstellung eine Ausführungsform der erfindungsgemäßen Entnahmevorrichtung,

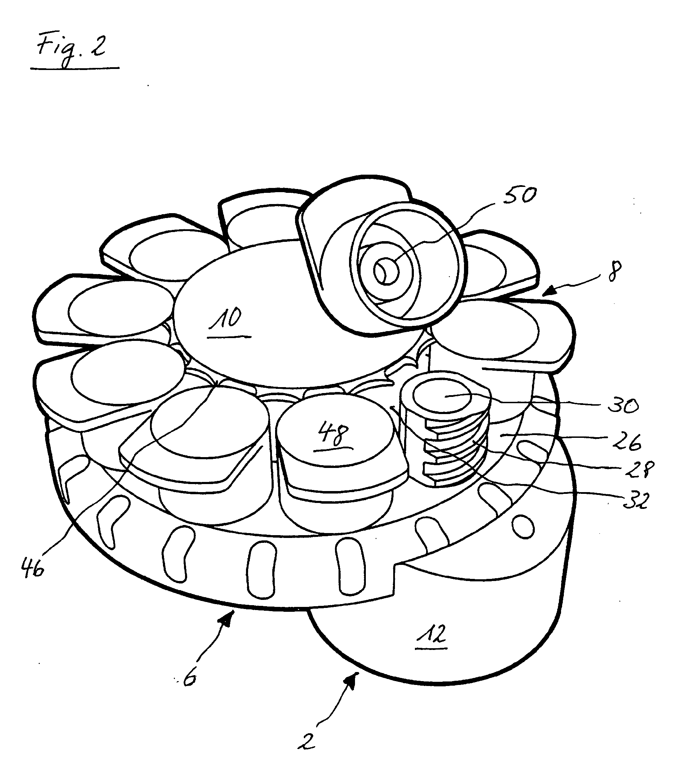

- Fig. 2: in perspektivischer Darstellung die Entnahmevorrichtung gemäß Fig. 1 im zusammengebauten Zustand und

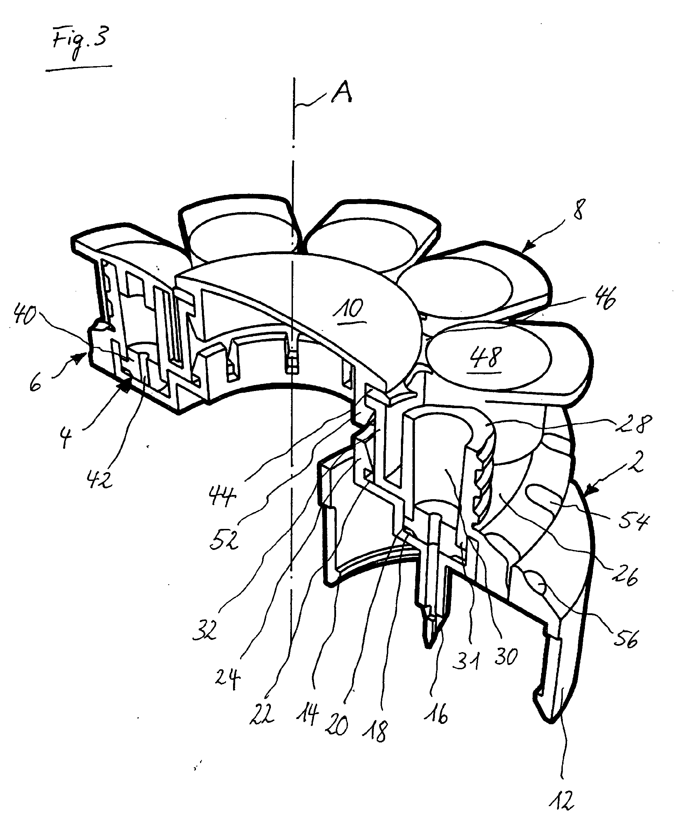

- Fig. 3: in teilgeschnittener perspektivischer Darstellung die Entnahmevorrichtung gemäß Fig. 2.

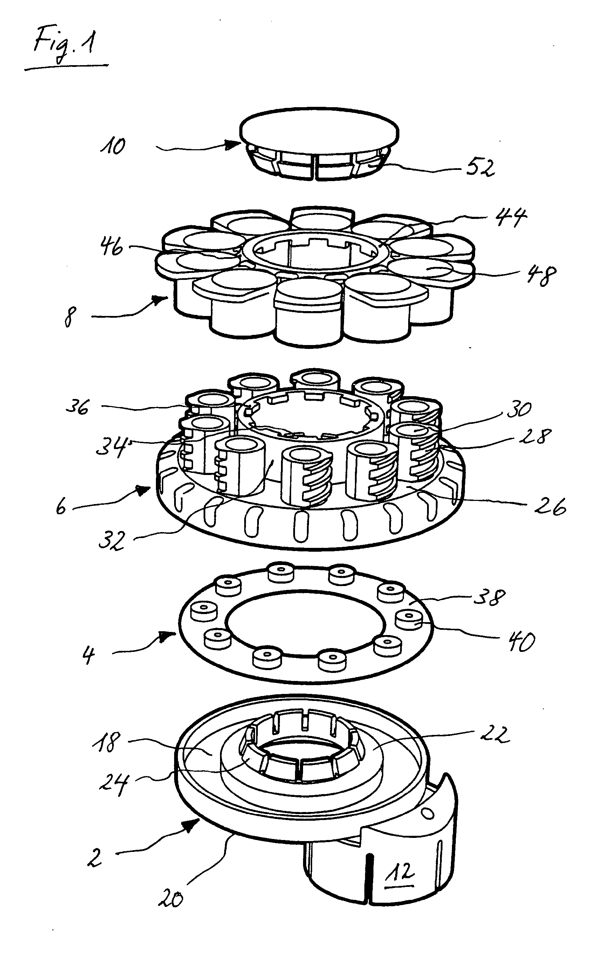

Die Figuren und insbesondere die Explosionsdarstellung in Fig. 1 zeigen, dass die erfindungsgemäße Entnahmevorrichtung im Wesentlichen aus einem Anschlussteil 2, einem Dichtring 4, einem Bauteil 6, einem Verschlusskappenteil 8 sowie einer Befestigungskappe 10 besteht.

Das als ein Kunststoff-Spritzgussteil ausgebildete Anschlussteil 2 bildet eine hülsenförmige Anschlusskappe 12 zum Anschließen der Entnahmevorrichtung an einen in den Figuren nicht dargestellten medizinischen Behälter, wie z.B. eine Infusionsflasche oder einen Kontrastmittelbehälter.

Dabei ist die Anschlusskappe 12 zum Anschluss an solche Behälter ausgebildet, die einen flaschenhalsartig verjüngten Entnahmebereich aufweisen, an dem eine Entnahmeöffnung von einer Membran verschlossen ist, die mittels einer die Außenwandung des Behälters in diesem Entnahmebereich wulstartig umgreifenden Aluminiummanschette mittels einer Crimpverbindung an dem Behälter befestigt ist.

Auf diesen Entnahmebereich des Behälters wird die Anschlusskappe 12, welche eine im Wesentlichen mit der Außenkontur des Entnahmebereichs korrespondierenden Innenkontur aufweist, aufgesetzt. Dabei umgreift ein im Bereich der offenen Stirnseite der Anschlusskappe 12 an dessen Innenwandung angeordneter Vorsprung 14, der sich über den gesamten Innenumfang der Anschusskappe 12 in Richtung dessen Mittelachse erstreckt, die Aluminiummanschette des medizinischen Behälters und ermöglicht so eine formschlüssige Befestigung der Anschlusskappe 12 und damit der Entnahmevorrichtung an dem Behälter.

Um den Vorsprung 14 an der Aluminiummanschette des Behälters vorbeiführen zu können, ist die Wandung der Anschlusskappe 12 in Richtung ihrer Längsausdehnung über den Umfang verteilt mehrfach geschlitzt, was in Verbindung mit einer gewissen Elastizität des Kunststoffmaterials der Anschlusskappe 12 zu einer Flexibilität der Wandung in radialer Richtung führt, wodurch der Innenquerschnitt der Anschlusskappe 12 im Bereich deren offener Stirnseite erweitert werden kann, um sich nach dem Passieren der Aluminiummanschette wieder zu seiner ursprünglichen Querschnittsgröße zurückzubilden.

Zum Durchstoßen der Membran des medizinischen Behälters und zur Herstellung einer Leitungsverbindung in den Behälter ist konzentrisch zu der Anschlusskappe 12 ein Hohldorn 16 angeordnet, dessen freies und spitztes Ende der offenen Seite der Anschlusskappe 12 zugewandt ist. Das andere Ende des Hohldorns 16 mündet in einer ebenen Grundfläche 18 einer Führung 20, in welcher das Bauteil 6 im montierten Zustand der Entnahmevorrichtung geführt ist.

Neben der Anschlusskappe 12 bildet auch die Führung 20 Teil des Anschlussteils 2. Sie ist an der von der offenen Seite der Anschlusskappe 12 abgewandten Stirnfläche dieser Anschlusskappe 12 angeordnet ist. Die Führung 20 ist derart exzentrisch zu der Anschlusskappe 12 angeordnet, dass sich die Anschlusskappe 12 und die daran angebundene Führung 20 nur in einem Teilabschnitt überlappen.

Die Grundfläche 18 der Führung 20 ist kreisringförmig ausgebildet und wird sowohl an ihrem Innen- als auch an ihrem Außenumfang von Seitenwandungen begrenzt, welche sich senkrecht zu der Grundfläche 18 in einer von der offenen Seite der Anschlusskappe 12 abgewandten Richtung erstrecken. Ausgehend von der inneren Seitenwandung der Grundfläche 18 erstreckt sich parallel zu der Grundfläche 18 eine weitere kreisringförmig ausgebildete Fläche 22 in Richtung einer Mittelachse A der Führung 20. Ausgehend von dem Innenumfang der Fläche 22 sind über den gesamten Umfang verteilt Rastelemente 24 angeordnet, welche senkrecht zu der Fläche 22 in eine von der offenen Seite der Anschlusskappe 12 abgewandten Richtung ausgerichtet sind.

Die Führung 20 dient zur Aufnahme und zum Halten des Bauteils 6, welches wie das Anschlussteil 2 als Kunststoff-Spritzgussteil ausgebildet ist. Das Bauteil 6 ist korrespondierend zu der Führung 20 ebenfalls kreisringförmig ausgebildet, wobei sich eine Außenwandung des Bauteils 6 zunächst in Richtung dessen Mittelachse A erstreckt und dann derart abknickt, dass sie eine Anschlussfläche 26 bildet, welche sich radial in Richtung der Mittelachse A des Bauteils erstreckt.

An der Anschlussfläche 26, die parallel zur Grundfläche 18 ist, sind über deren Umfang verteilt zehn Anschlüsse 28 mit zu der Mittelachse A des Bauteils 6 parallelen Längsachsen angeordnet. Diese Anschlüsse 28 weisen jeweils einen zu der Mittelachse A des Bauteils 6 parallelen Strömungskanal 30 auf. Die Strömungskanäle 30 sind dabei durch die die Anschlussfläche 26 bildende Wandung des Bauteils 6 geführt und an der von der Anschlussfläche 26 abgewandten Stirnseite der Wandung durch rohrförmige Stutzen 31 verlängert. Die Anschlüsse 28 bilden die Flüssigkeitsauslässe der Entnahmevorrichtung und sind jeweils als Luerlockanschluss ausgebildet, so dass die Anschlüsse 28 mit einem dementsprechend ausgebildeten Luerlockanschluss z. B. mit einer Spritze oder mit einer Schlauchleitung verbindbar sind.

Ausgehend von der der Mittelachse A des Bauteils 6 zugewandten Innenkante der Anschlussfläche 26 erstreckt sich zu dieser Anschlussfläche 26 senkrecht eine Wandung 32 in Richtung der Längsausdehnung der Anschlüsse 28. Dabei sind an der Innenumfangsfläche dieser Wandung 32 jeweils über den gesamten Umfang verteilt im Bereich der von der Anschlussfläche 26 und der Wandung 32 gebildeten Kante Rastelemente 34 und im Bereich des freien Endes der Wandung 32 Rastelemente 36 angeordnet, wobei sowohl die Rastelemente 34 als auch die Rastelemente 36 in Richtung der Mittelachse des Bauteils 6 ragen.

Im zusammengebauten Zustand der Entnahmevorrichtung ist zwischen der Führung 20 des Anschlussteils 2 und dem Bauteil 6 der Dichtring 4 angeordnet. Der Dichtring 4 ist ein Gummidichtring mit einem flachen kreisringförmigen Grundkörper 38, dessen radiale Abmessungen im Wesentlichen den Abmessungen der Grundfläche 18 der Führung 20 entsprechen. An einer der Stirnflächen des Grundkörpers 38 erstrecken sich zehn noppenförmige Erhöhungen 40 senkrecht zu der Stirnfläche. Die Erhöhungen 40 besitzen eine im Wesentlichen zylindrische Form. Der Durchmesser der Erhöhungen 40 ist komplementär zu den Innendurchmessern der in den Anschlüssen 28 des Bauteils 6 angeordneten Strömungskanäle 30. Jede der Erhöhungen 40 wird durch einen in deren Längsrichtung verlaufenden Strömungskanal 42 durchbrochen.

Bei einem mit dem Dichtring 4 und dem Bauteil 6 montierten Anschlussteil 2, greifen die Erhöhungen 40 des Dichtringes 4 in die rohrförmigen Stutzen 31 des Bauteils 6 ein. Die ebene und flächige Stirnseite des Grundkörpers 38 des Dichtrings 4 liegt dann auf der Grundfläche 18 der Führung 20 auf. Die Außenwandung des Bauteils 6 liegt mit ihrer Innenseite an der Außenseite der äußeren, die Grundfläche 18 begrenzenden Wandung der Führung 20 an. Dabei hintergreift diese die Grundfläche 18 nach außen begrenzende Wandung die Stutzen 31 des Bauteils 6, die hierzu diametral entgegengesetzt auch mit der Außenseite der inneren, die Grundfläche 18 begrenzenden Wandung der Führung 20 zur Anlage kommen. Im Bereich zwischen den Anschlüssen 28 und der Wandung 32 liegt die Wandung des Bauteils 6, welche die Anschlussfläche 26 bildet mit ihrer dazu entgegengesetzten Seite auf der Fläche 22 der Führung 20 auf. Die formschlüssige Befestigung des Bauteils 6 an der Führung 20 bzw. an dem Anschlussteil 2 erfolgt durch Hintergreifen der Rastelemente 24 der Führung 20 durch die Rastelemente 34 des Bauteils 6. Dieser Formschluss gewährleistet zum einen den Zusammenhalt von Anschlussteil 2 und Bauteil 6 und ermöglicht zum anderen eine Bewegung des Bauteils 6 mit dem daran angeordneten Dichtring 4 in der Führung 20 des Anschlussteils 2.

Das Verschlusskappenteil 8 besteht aus einem inneren Ring 44, an dessen Außenumfang jeweils über einen dünnen Steg 46 zehn Verschlusskappen 48 angebunden sind. Die Stege 46 bilden Sollbruchstellen, an denen die einzelnen Verschlusskappen 48 von dem Ring 44 gelöst werden können.

Im montierten Zustand ist das Verschlusskappenteil 8 derart angeordnet, dass die Verschlusskappen 48 die Anschlüsse 28 des Bauteils 6 überdecken. Dabei greift jeweils ein in dem Innenlumen der Verschlusskappen 48 angeordneter zylindrischer Zapfen 50, dessen Außenabmessungen mit den Innenabmessungen eines Strömungskanals 30 in dem Anschluss 28 korrespondieren, in einen Strömungskanal 30 ein. Auf diese Weise werden die Fluidauslässe der Anschlüsse 28 keimdicht verschlossen. Der Ring 44 des Verschlusskappenteils 8 liegt im montierten Zustand auf dem freien Ende der Wandung 32 des Bauteils 6 auf.

Die Befestigung des Verschlusskappenteils 8 an dem Bauteil 6 erfolgt mit der Befestigungskappe 10. Die Befestigungskappe 10 bildet einen kreisförmigen Deckel, wobei an einer Stirnseite des Deckels in Nähe des Au-βenumfangs Rastelemente 52 angeordnet sind die sich normal zu der Stirnfläche erstrecken. Die Befestigungskappe 10 wird so in die von der Wandung 32 des Bauteils 6 gebildete Öffnung geschoben, dass die Rastelemente 52 der Befestigungskappe 10 die Rastelemente 36, die an der Innenseite der Wandung 32 angeordnet sind, hintergreifen. Auf diese Weise wird der auf dem freien Ende der Wandung 32 aufliegende Ring 44 des Verschlusskappenteils 8 zwischen der Wandung 32 des Bauteils 6 und dem Deckel der Befestigungskappe 10 festgelegt.

Zur Entnahme einer Flüssigkeit aus einem medizinischen Behälter wird die Anschlusskappe 12 des Anschlussteils 2 derart über den Entnahmebereich des Behälters gesteckt, dass der Dorn 16 die Membran des Behälters durchstößt und der Vorsprung 14 der Anschlusskappe 12 die Aluminiummanschette zur Befestigung der Membran an dem Behälter hintergreift und so die Entnahmevorrichtung an dem Behälter festlegt.

Vor der Entnahme aus dem Behälter ist das Bauteil 6 und der damit verbundene Dichtring 4 so in der Führung 20 des Anschlussteils 2 verdreht, dass der Strömungspfad des Hohldorns 16 von dem Grundkörper 38 des Dichtrings 4 verschlossen wird, so dass keine Flüssigkeit aus dem Behälter auslaufen kann.

In diesem Zustand wird eine der Verschlusskappen 48 von einem Anschluss 28 des Bauteils 6 abgezogen und abgebrochen. An dem dann an diesem Anschluss 28 freiliegenden Luerlock kann nun ein Schlauch mit einem entsprechenden Luerlockanschluss befestigt werden.

Daraufhin wird das Bauteil 6 und der Dichtring 4 zum Anschlussteil 2 so gedreht, dass der Strömungspfad durch den Hohldorn 16, der Strömungskanal 42 des Dichtrings 4 sowie der in dem betreffenden Anschluss 28 ausgebildete Strömungskanal 30 eine Fluidleitung von dem medizinischen Behälter zu dem angeschlossenen Schlauch bilden, über welchen die Flüssigkeit dann einem Patienten zugeführt werden kann. Die hierfür erforderliche Verdrehposition des Bauteils 6 gegenüber dem Anschlussteil 2 wird durch Markierungen 54 an dem Bauteil 6, welche jedem der Anschlüsse 28 zugeordnet sind sowie eine Markierung 56 an dem Anschlussteil 2 angezeigt, wobei die betreffende Markierung 54 und die Markierung 56 zur Herstellung einer Leitungsverbindung in radialer Richtung des Anschlussteils 2 zur Deckung gebracht werden müssen.

Zur Beendigung der Flüssigkeitsentnahme aus dem Behälter wird das Bauteil 6 gegenüber dem Anschlussteil 2 so verdreht dass die Markierung 56 und die Markierung 54 zueinander radial versetzt sind. In dieser Position verschließt der Dichtring 4 den Strömungspfad durch den Hohldorn 16, so dass keine Flüssigkeit aus dem Behälter austreten kann und von außen keine Keime oder Verunreinigungen über das Bauteil 6 in den Behälter gelangen können. Der nun benutzte Anschluss 28 des Bauteils 6 ist durch die fehlende Verschlusskappe 48 erkennbar. Zur erneuten Verwendung der Entnahmevorrichtung wird eine weitere Verschlusskappe 48 von einem Anschluss 38 abgezogen und der oben beschriebene Vorgang wiederholt.

In den Figuren nicht dargestellt können in einem Kontaktbereich zwischen dem Anschlussteil 2 und dem Bauteil 6 keilförmige Rastelemente vorgesehen sein, deren Ausbildung die Bewegung des Bauteils 6 in der Führung 20 nur in eine Richtung, d. h. im Uhrzeiger- oder im Gegenuhrzeigersinn gestattet. In Verbindung mit einer Anschlagspaarung, bestehend aus einem an dem Bauteil 6 angeordneten Anschlag und einem beispielsweise an der Führung 20 korrespondierend angeordneten zweiten Anschlag kann eine über einen Winkel von 360° hinausgehende Drehbewegung des Bauteils 6 in der Führung 20 ausgeschlossen werden, wodurch einer Mehrfachbenutzung der Anschlüsse 28 weiter vorgebeugt wird.

### Bezugszeichenliste

- 2: Anschlussteil
- 4: Dichtring
- 6: Bauteil
- 8: Verschlusskappenteil
- 10: Befestigungskappe
- 12: Anschlusskappe
- 14: Vorsprung
- 16: Hohldorn
- 18: Grundfläche
- 20: Führung
- 22: Fläche
- 24: Rastelement
- 26: Anschlussfläche
- 28: Anschluss
- 30: Strömungskanal
- 31: Stutzen
- 32: Wandung
- 34: Rastelement
- 36: Rastelement
- 38: Grundfläche
- 40: Erhöhung
- 42: Strömungskanal
- 44: Ring
- 46: Steg
- 48: Verschlusskappe
- 50: Zapfen
- 52: Rastelement
- 54: Markierung
- 56: Markierung
- A: Mittelachse

## Patentansprüche

1. Vorrichtung zur Entnahme von Flüssigkeit aus einem medizinischen Behälter mit einem Anschlussteil (2), welches zum Anschluss an den Behälter und zur Herstellung einer Leitungsverbindung in den Behälter ausgebildet ist und mit einem dazu beweglich gelagerten Bauteil (6), das eine Vielzahl von Anschlüssen (28) für einen Flüssigkeitsauslass aufweist, wobei das Anschlussteil (2) und das Bauteil (6) so zueinander bewegbar sind, dass eine Leitungsverbindung zwischen Behälter und wahlweise zumindest einem der Anschlüsse (28) gebildet ist.

2. Entnahmevorrichtung nach einem der vorangehenden Ansprüche, wobei das Bauteil (6) in dem Anschlussteil (2) formschlüssig geführt und gehalten ist.

3. Entnahmevorrichtung nach einem der vorangehenden Ansprüche, wobei die Anschlüsse (28) an dem Bauteil (6) in Richtung der Relativbewegung von Anschlussteil (2) und Bauteil (6) in Reihe angeordnet sind.

4. Entnahmevorrichtung nach einem der vorangehenden Ansprüche, wobei das Bauteil (6) kreisringförmig ausgebildet ist und wobei die Anschlüsse (28) über den Umfang des Kreisringes verteilt angeordnet sind.

5. Entnahmevorrichtung nach einem der vorangehenden Ansprüche, wobei zwischen dem Anschlussteil (2) und dem Bauteil (6) eine Dichtung (4) vorgesehen ist, welche das Anschlussteil (2) gegenüber dem Bauteil (6) flüssigkeits- und keimdicht abdichtet.

6. Entnahmevorrichtung nach Anspruch 5, wobei die Dichtung (4) von einem Gummiring gebildet wird und wobei der Gummiring an einer Stirnfläche noppenförmige Erhöhungen (40) aufweist, welche jeweils einen Strömungskanal (42) bilden und in die Anschlüsse (28) formschlüssig eingreifen.

7. Entnahmevorrichtung nach einem der vorangehenden Ansprüche, wobei das Anschlussteil (2) auf der vom Bauteil (6) abgewandten Seite einen Hohldorn (16) aufweist.

8. Entnahmevorrichtung nach einem der vorangehenden Ansprüche, wobei zumindest zwei Anschlüsse vorgesehen sind, welche vorzugsweise verriegelbar ausgebildet sind und insbesondere sich hinsichtlich ihrer Form und/oder Abmessung unterscheiden.

9. Entnahmevorrichtung nach einem der vorangehenden Ansprüche, wobei die Anschlüsse (28) jeweils als Luerlockanschluss ausgebildet sind.

10. Entnahmevorrichtung nach einem der vorangehenden Ansprüche, wobei an jedem der Anschlüsse (28) ein keimdichter vorzugsweise nach dem Entfernen nicht wieder einsetzbarer Verschluss vorgesehen ist.

11. Entnahmevorrichtung nach Anspruch10, wobei die Verschlüsse von einer Siegelfolie gebildet sind.

12. Entnahmevorrichtung nach Anspruch 10, wobei die Verschlüsse von Verschlusskappen (48) gebildet sind.
